(19) Europäisches Patentamt / European Patent Office / Office européen des brevets

(11) **EP 2 246 055 B1**

(12) **EUROPEAN PATENT SPECIFICATION**

(45) Date of publication and mention of the grant of the patent:
**01.06.2016 Bulletin 2016/22**

(21) Application number: **09709122.7**

(22) Date of filing: **15.01.2009**

(51) Int Cl.:
***A61K 31/495*** *(2006.01)* ***A61K 31/496*** *(2006.01)*
***A61K 31/506*** *(2006.01)* ***A61P 25/04*** *(2006.01)*
***A61P 43/00*** *(2006.01)*

(86) International application number:
**PCT/CN2009/070162**

(87) International publication number:
**WO 2009/097774 (13.08.2009 Gazette 2009/33)**

(54) **THE USE OF ARYL PIPERAZINE DERIVATIVES IN MANUFACTURING MEDICANTS FOR TREATING PAIN**

VERWENDUNG VON ARYLPIPERAZINDERIVATEN BEI DER HERSTELLUNG VON ANALGETIKA

UTILISATION DE DÉRIVÉS D'ARYL PIPÉRAZINE DANS LA FABRICATION DE MÉDICAMENTS POUR TRAITER LA DOULEUR

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **31.01.2008 CN 200810018896**

(43) Date of publication of application:
**03.11.2010 Bulletin 2010/44**

(73) Proprietor: **NHWA Pharma.corporation**
**Jiangsu 221007 (CN)**

(72) Inventors:
- **ZHANG, Guisen**
**Xuzhou**
**Jiangsu 221007 (CN)**
- **GUO, Lin**
**Xuzhou**
**Jiangsu 221007 (CN)**
- **YANG, Xiangping**
**Xuzhou**
**Jiangsu 221007 (CN)**
- **XU, Xiangqing**
**Xuzhou**
**Jiangsu 221007 (CN)**
- **LI, Jianqi**
**Shanghai 200040 (CN)**
- **WANG, Guan**
**Shanghai 200040 (CN)**
- **MA, Yanqin**
**Xuzhou**
**Jiangsu 221007 (CN)**
- **HU, Shuming**
**Xuzhou**
**Jiangsu 221007 (CN)**
- **LIU, Shicheng**
**Xuzhou**
**Jiangsu 221007 (CN)**
- **ZHOU, Shixia**
**Xuzhou**
**Jiangsu 221007 (CN)**

(74) Representative: **V.O.**
**P.O. Box 87930**
**2508 DH Den Haag (NL)**

(56) References cited:
**WO-A1-2007/071055**
**WO-A2-2007/104783**
**US-A- 5 369 103**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 246 055 B1

**Description**

## FIELD OF THE INVENTION

The invention relates to the use of aryl piperazine derivatives and pharmaceutically acceptable salts thereof alone or in combination with other analgesics in manufacturing a medicament for treating acute pain, neuropathic pain or receptive nociceptive pain in mammals, including human beings.

## BACKGROUND OF THE INVENTION

EP1627871 discloses a compound of the following formula, which can be used as an intermediate to prepare a steroid hormone sulfatase inhibitor:

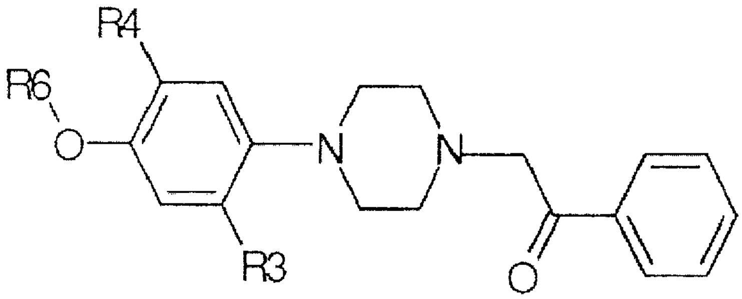

wherein R3 and R4 each represent hydrogen, halogen, cyano, or lower alkyl: R6 represents hydrogen or a protecting group of hydroxyl. The publication does not mention the pharmacological activity of the compound of the above formula.
EP1123926 discloses a compound of the following formula, which can be used as an intermediate to produce piperazine derivatives for treatment of central nervous system dysfunction:

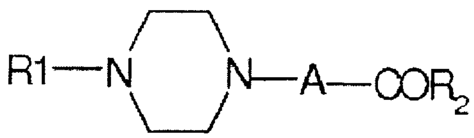

wherein R1 is monocyclic or dicyclic aryl or heteroaryl; R2 is aryl or heteroaryl; A is $CH_2$ or $-CH_2CH_2-$. The publication does not mention the pharmacological activity of the compound of the above formula.
WO 2007/071055 describes that quinoline and quinazoline compounds modulate the activity of gated ion channels and are useful in the treatment of diseases and disorders related to pain, inflammation, the neurological system, the gastrointestinal system and the genitourinary system. Amongst others, the compound 1-(4-methoxyphenyl)-2-[4-(2-methyl-quinolin-4-yl)-piperazin-1-yl]-ethanone is disclosed in WO 2007/071055 for the treatment of pain.
WO 2007/104783 describes 1,4-disubstituted 3-cyano-pyridone derivatives according to formula (I) defined therein and their use as positive allosteric modulators of mGluR2-receptors. The 1,4-disubstituted 3-cyano-pyridone derivatives are stated to be useful for treating or preventing various necrological and psychiatric disorders associated with glutamate dysfunction, including pain.
Li, J. et al., "Design and synthesis of aralkyl-ketone piperazine derivatives and their antalgic activities", Acta Pharmaceutica Sinica 2007, 42(11), 1166-1175, describes the synthesis of aralkyl-ketone piperazine derivatives. A mice writhing model, a rat hot plate model and a rat tail flick model was employed to evaluate the analgesic biological activities of the compounds tested.
It has been established that compounds of the following formula (I) and pharmaceutically acceptable salts thereof can be used alone or in combination with other analgesics (in this case synergetic effect can occur) for treating acute pain, neuropathic pain or receptive nociceptive pain in mammals, including human beings.

## SUMMARY OF THE INVENTION

The invention provides use of aryl piperazine derivatives of formula (I) and pharmaceutically acceptable salts

thereof for the manufacture of a medicament for treating acute pain, neuropathic pain or receptive nociceptive pain in mammals, including human beings:

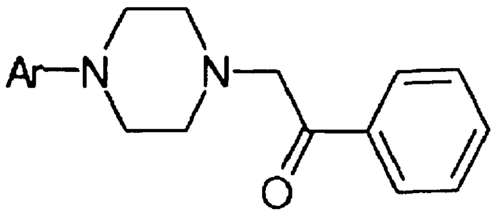

( I )

wherein Ar is phenyl monosubstituted or disubstituted with F. Cl, Br, methyl, methoxyl or trifluoromethyl; or is an unsubstituted, monosubstituted or disubstituted heteroaryl, wherein the heteroaryl is selected from benzothiazolyl, pyridyl, or pyrimidinyl and the substituent(s) are selected from the group consisting of halogen, $C_{1-6}$, alkyl, $C_{1-4}$ alkoxyl and halogenated $C_{1-4}$ alkyl.

According to another aspect of the invention, the compound of formula (I) is provided for use in the treatment of acute pain, neuropathic pain or receptive nociceptive pain in mammals, including human beings.

## DETAILED DESCRIPTION OF THE EMBODIMENTS

In the present disclosure, halogen refers to F, Cl, Br or I. preferably F, Cl or Br.

$C_{1-6}$ alkyl means a linear, branched or cyclic saturated alkyl containing 1, 2, 3, 4, 5 or 6 carbon atoms, such as methyl, ethyl, propyl, isopropyl, n-butyl, n-pentyl, and n-hexyl, Methyl is preferable.

$C_{1-4}$ alkoxyl means an alkyl-O- group, wherein the alkyl is an alkyl containing 1, 2, 3 or 4 carbon atoms as defined above, such as methoxyl, ethoxyl, n-propoxyl, iso-propoxyl and n-butoxyl, Methoxyl is preferable.

Halogenated $C_{1-4}$ alkyl means an alkyl containing 1, 2, 3 or 4 carbon atoms as defined above which is substituted partly or completely by halogen. Trifluoromethyl is preferable.

Aryl means an aliphatic aromatic group containing 5-14 carbon atoms, such as phenyl or naphthyl. Heteroaryl means a monocyclic or bicyclic 5-14 member aryl containing 1-4, preferably 1-2 heteroatoms selected from O, N and S.

As noted herein above, the Ar group in formula (I) is mono- or di-substituted phenyl, wherein the substituent is selected from F, Cl, Br, methyl, methoxyl and trifluoromethyl; or is an unsubstituted, monosubstituted or disubstituted heteroaryl, wherein the heteroaryl is selected from benzothiazolyl, pyridyl or pyrimidinyl and the substituent(s) are selected from the group consisting of halogen, $C_{1-6}$ alkyl, $C_{1-4}$ alkoxyl and halogenated $C_{1-4}$ alkyl.

In one embodiment of the present invention, the heteroaryl represents benzothiazolyl, pyridyl or pyrimidinyl, and the substituted heteroaryl comprises no more than two substituents selected from F, Cl, Br, methyl, methoxyl and trifluoromethyl.

The compound of formula (I) for use in accordance with the present invention may be prepared in the form of pharmaceutically acceptable salts according to common methods known in the art. The pharmaceutically acceptable salts are those conventionally used in the art, such as acid addition salts. The pharmaceutically acceptable acid addition salts include inorganic acid addition salts made from inorganic acids such as hydrochloric acid, hydrobromic acid, phosphoric acid or sulfuric acid; organic acid addition salts made from organic acids such as acetic acid, propionic acid, lactic acid, citric acid, fumaric acid, malic acid, succinic acid, salicylic acid, maleic acid, glycerophosphoric acid, acetosalicylic acid, and alkyl or aryl sulfonic acid such as methanesulfonic acid, benzene sulfonic acid or toluene sulfonic acid.

The use of the compound of formula (I) or the pharmaceutically acceptable salts for the manufacture of a medicament for treating pain refers to the manufacture of an analgesic for use in the clinic. The acute pain includes, but is not limited to, headache, arthralgia, muscular tension or dysmenorrheal. The neuropathic pain includes, but is not limited to, chronic backache, postherpetic neuralgia, diabetic peripheral neuralgia, fibromyalgia, cancer-related pain, phantom limb pain, labour pains and opioid analgesics resistant neuralgia. The perceptive nociceptive pain includes, but is not limited to, postoperative pain, toothache, pain from surgery, and pain from serious burns.

The compounds of formula (I) for use in accordance with the present invention can be formulated to a pharmaceutical composition according to a common standard pharmaceutical technique for administration of the compounds in the treatment. For example, the compounds of formula (I) may be dissolved in oil, propylene glycol or other solvents often used for preparing injections. Preferable carriers are, but are not limited to, a saline solution, polyethylene glycol, ethanol, vegetable oil, isopropyl myristate, and the like. For topical administration, the compounds of formula (I) may be formulated into the form of ointment or cream.

The compounds of formula (I) may be dissolved, suspended or emulsified in an aqueous solution such as a conventional saline solution and 5% glucose solution, or a non-aqueous solution such as synthetic fatty glyceride, high fatty acids ester and propylene glycol, and thereby an injection is formulated. The composition may comprise conventional additives, such as a solubilizing agent, an isotonizing agent, a suspending agent, an emulsifying agent, a stabilizing agent and a preservative.

The preferable administration dose of the compound of formula (I) may vary depending on such factors as the condition and the weight of patients, severity of the disease, formulation of the medicament, as well as the route and time of administration, but those skilled in the art will be able to determine the dose accordingly. To obtain a preferable effect, however, the daily dosage of the compounds of formula (I) may be 1-200 mg/kg (body weight), preferably 1-100 mg/kg (body weight), while the dose may be administered once or in several times.

According to the method of administration, the pharmaceutical composition may contain 0.001-99 wt% of the compound of formula (I), preferably 0.01-60 wt%.

The pharmaceutical composition may be administered to mammals, including rats, mice, domestic animals or human beings by various routes. Any administration route can be applied, such as oral, transdermal, parenteral, subcutaneous, nasal, intramuscular or intravenous administration.

According to another embodiment of the invention, the compound of formula (I) may also be employed together with other analgesics so as to obtain a synergetic effect, with a ratio there between being one part of the compound of formula (I) to 1-10 parts of the other analgesics. The other analgesics include, but are not limited to, non-steroidal anti-inflammatory drugs such as aspirin and naproxen, narcotic analgesics such as morphine, dolantin and fentanyl.

The compound of formula (I) may be manufactured according to the following scheme:

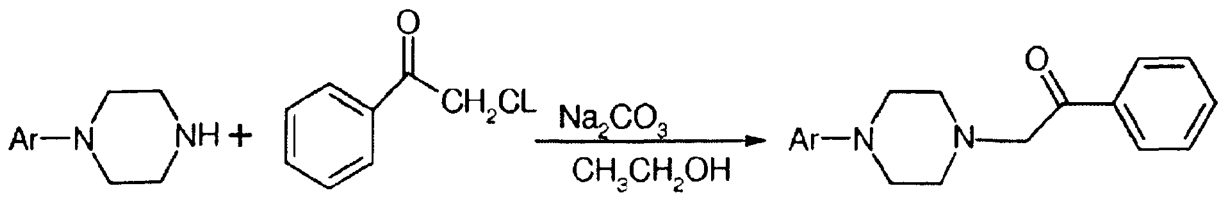


For example, 0.24 mol of a substituted phenyl piperazine or benzothiazoyl-, pyridyl- or pyrimidinyl-piperazine, 0.24 mol of α-chloro acetophenone. 480 ml of ethanol, and 50.4 g of sodium carbonate are added to a 1000 ml flask with four necks. After the mixture is refluxed while stirring for 4 hours, the reaction is monitored with thin-layer chromatography until the sample point of the principal product does not change any more, which indicates that the reaction is complete. After heat filtration, the filtrate is evaporated to dryness, and the residue is dissolved with methylene chloride. The methylene chloride solution is washed with water until the pH is close to neutral. After being dried with anhydrous sodium sulfate, the pH of the solution is adjusted to 3-4 with a saturated hydrogen chloride solution in ethyl acetate. The solid is filtrated, washed with ether, recrystallized with anhydrous ethanol, and then dried under vacuum at 50 °C. An off-white solid is obtained.

## Examples

The present invention is further explained by the following examples, but those skilled in the art will understand that the examples are only intended to explain the invention and should not be construed as limiting the scope of the present invention.

### Preparation examples

The compounds shown in the following Table 1 are prepared according to the above conventional process from corresponding starting materials.

Table 1

| |
|---|
| 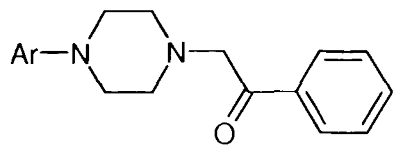 |

(continued)

| Example | Ar | HNMR(300MHz), δ (solvent) | MS (m/z) |
|---|---|---|---|
| 1 | 2-methoxyl phenyl | 7.95(d,J=7.2,2H), 7.72(t,J=7.2,1H) 7.55(t,J=7.6,2H), 7.16(t,J=7.6,1H) 7.12(d,J=7.6,1H), 7.05(d,J=7.6,1H) 7.01(t,J=7.6,1H), 5.00(s,1H) 3.82(s,3H), ($CDCL_3$) | 310.3$[M]^+$ 205.2$[C_{12}H_{17}NaO]^+$ 105.1 $[C_7H_5O]^+$ |
| 2 | 3-methoxyl phenyl | 8.01(d,J=7.6,2H), 7.64 (t,J=7.6,1H) 7.53(t,J=7.6,2H), 7.10 (t,J=8.0,1H) 6.52(dd,J=2.0,7.6,1H),6.44(t,J=2.0,1H) 6.36(dd,J=2.0,7.6,1H), 3.88(s,2H). 3.71(s,3H),($CDCL_3$) | 310.3$[M]^+$ 205.2$[C_{12}H_{17}N_2O]^+$ 105.1 $[C_7H_5O]^+$ |
| 3 | 4-methoxyl phenyl | 7.94(d,J=7.6,2H), 7.72(t,J=7.6,1H) 7.55(t,J=7.6,2H), 7.12(d.J=8.8,2H) 7.08(d,J=8.8,2H), 5.00(s,2H) 3.76 (s,3H), ($CDCL_3$) | 310.3$[M]^+$ 205.2$[C_{12}H_{17}N_2O]^+$ 105.1$[C_7H_5O]^+$ |
| 4 | 2-chloro phenyl | 8.02(d,J=7.6,2H), 7.64 (t,J=7.6,1H) 7.53(t,J=7.6,2H), 7.39 (dd,J=1.2,7.6,1H) 7.28(td,J=1.2,7.6.1H),7.16(dd,J=1.2,7.6, 1H), 7.03(td,J=1.2,7.6,1H), 3.93 (s,2H) ($CDCL_3$) | 314.1$[M]^+$ 209.0 $[C_{12}H_{17}N_2CL]^+$ 105.1 $[C_7H_5O]^+$ |
| 5 | 3-chloro phenyl | 8.01(d,J=7.6,2H), 7.64(t,J=7.2,1H) 7.53(t,J=8.0,2H), 7.20(t,J=8.0, 1H) 6.93(t,J=2.0, 1H),6.89(dd,J=1.2,7.6,1H) 6.77(dd,J=1.2,7.6,1H), 3.92(s,2H) ($CDCL_3$) | 314.1$[M]^+$ 209.0 $[C_{12}H_{17}N_2CL]^+$ 105.1 $[C_7H_5O]^+$ |
| 6 | 4- chloro phenyl | 8.01(d,J=7.6,2H), 7.64(t,J=7.2,1H) 7.53(t,J=7.6,2H), 7.22(d,J=8.8, 2H) 6.94(d,J=8.8,2H), 3.92(s,2H), ($CDCL_3$) | 314.1$[M]^+$ 209.0 $[C_{12}H_{17}CLN_2]^+$ 105.1 $[C_7H_5O]^+$ |
| 7 | 2,3-dichloro phenyl | 8.01 (d,J=7.6,2H), 7.59(tt,J=1.2,7.6,1H) 7.53(t,J=7.6,2H), 7.17-7.13(m, 2H) 6.97(dd,J=5.6,2.8,1H), 3.92(s,2H) ($CDCL_3$) | 348.3 $[M]^+$ 243.2 $[C_{12}H_{13}CL_2N_2]^+$ 105.1 $[C_7H_5O]^+$ |
| 8 | 4-fluorophenyl | 8.01(d,J=7.2,2H), 7.64(t,J=7.2,1H) 7.53(t,J=7.6.2H), 7.04(t,J=8.8, 2H) 6.95-6.93(m,2H), 3.92(s,2H), ($CDCL_3$) | 298.1 $[M]^+$ 193.0 $[C_{12}H_{13}FN_2]^+$ 105.1 $[C_7H_5O]^+$ |
| 9 | 3- trifluoro methylphenyl | 7.99 (d,J=7.2,2H), 7.58(t,J=7.6,1H) 7.43(t,J=7.6,2H), 7.32(t,J=8.0,1H) 7.14(br, 1H), 7.04(d,J=8.0,1H) 7.01 (t,J=7.6,1H), 4.93(s,2H) 3.79-3.14(br,3H), ($CDCL_3$) | 348.3 $[M]^+$ 243.2 $[C_{12}H_{14}F_3N_2]^+$ 105.1 $[C_7H_5O]^+$ |
| 10 | 2.3-dimethyl phenyl | 7.92(d,J=7.2,2H), 7.59(t,J=7.6,1H) 7.45(t,J=7.6,2H), 6.94(t,J=7.6,1H) 6.86(d,J=7.6,1H), 6.82(d,J=7.6,1H) 4.94 (s,2H), 3.59-3.09(br,3H). ($CDCL_3$) | 308.3 $[M]^+$ 203.2 $[C_{13}H_{19}N_2]^+$ 105.1 $[C_7H_5O]^+$ 132.2 $[C_9H_{10}N]^+$ |
| 11 | 6-methoxyl-2-benzothiazolyl | 7.92(d,J=7.6,2H), 7.58(tt,J=1.2,7.2,1H) 7.47 (t,J=7.6,2H),7.15(t,J=2.4,1H) 7.45(s,1H), 3.90(s,2H), 6.90 (dd,J=2.4,7.6,1H), 3.68((t,J=5.2,4H) 3.82 (s,2H), ($CDCL_3$) | 367.3 $[M]^+$ 262.2 $[C_{13}H_{16}N_3OS]^+$ 105.1 $[C_7H_5O]^+$ 207.2 $[C_{10}H_{11}N_2OS]^+$ |

(continued)

| Example | Ar | HNMR(300MHz), δ (solvent) | MS (m/z) |
|---|---|---|---|
| 12 | 6-methyl-2-benzothiazolyl | 8.00 (d,J=7.6,2H), 7.64(t,J=7.6,1H) 7.56-7.51 (m,3H), 7.34(d,J=8.4,1H) 7.08(dd,J=0.8,7.6,1H), 3.97(s,2H) 3.55 (t, J=1.2,2H), ($CDCL_3$) | 351.3 $[M]^+$ 246.2 $[C_{13}H_{16}N_3S]^+$ 105.1 $[C_7H_5O]^+$ 191.2 $[C_{10}H_{11}N_2S]^+$ |
| 13 | 4-methyl-2-benzothiazolyl | 8.00(m,2H), 7.58 (tt,J=1.2,7.2,1H) 7.49 -7.43(m,3H), 6.98(t,J=7.6,1H) 7.12(d,J=7.2,1H), 3.74(t,J=5.2,4H) 3.91(s,2H), ($CDCL_3$) | 351.3 $[M]^+$ 246.2 $[C_{13}H_{16}N_3S]^+$ 105.1 $[C_7H_5O]^+$ 191.2 $[C_{10}H_{11}N_2S]^+$ |
| 14 | 6-chloro-2-benzothiazolyl | 7.98 (d,J=7.6,2H 7.60(tt,J=1.2,7.2,1H) 7.57 (d,J=2.0,1H), 7.48 (t,J=7.6,2H) 7.45 (d,J=8.8,1H), 7.25 (dd,J=2.0,8.8,1H) 3.97(s,2H), ($CDCL_3$) | 371.3 $[M]^+$ 266.2 $[C_{12}H_{14}CLN_3S]^+$ 105.1 $[C_7H_5O]^+$ 211.2 $[C_9H_8LN_2S]^+$ |
| 15 | 4-chloro-2-benzothiazolyl | 7.98 (d,J=7.6,2H), 7.59(t,J=7.6,1H) 7.49 -7.42 (br,3H), 7.31(dd,J=1.2,7.6,1H) 6.98(t,J=8.0,1H), 3.97(s,2H) 3.72 (t,J=5.2,4H), ($CDCL_3$) | 371.3 $[M]^+$ 266.2 $[C_{12}H_{14}CLN_3S]^+$ 105.1 $[C_7H_5O]^+$ 211.2 $[C_9H_8LN_2S]^+$ |
| 16 | 2-pyrimidinyl | 8.51 (d,J=5.2,2H), 7.91 (d,J=7.6,2H) 7.68 (t,J=7.6,2H), 7.51 (t,J=8.0,1H) 6.97 (t,J=5.2,1H) 5.00(s,2H) 4.14-2.89 (br,8H), ($CDCL_3$) | 282.2 $[M]^+$ 177.1 $[C_9H_{13}N_4]^+$ 105.1 $[C_7H_5O]^+$ |
| 17 | 6-chloro-2-pyridyl | 8.01(d,J=7.6,2H), 7.64 (t,J=7.6,1H) 7.53(t?J=7.6,2H), 7.10 (t,J=8.0,1H) 6.52(dd,J=2.0,7.6,1H),6.44(t,J=2.0,1H) 6.36(dd,J=2.0,7.6,1H), 3.88(s,2H), 3.71(s,3H),($CDCL_3$) | 315.2 $[M]^+$ 210.1 $[C_9H_{12}CLN_3]^+$ 105.1 $[C_7H_5O]^+$ |

The analgesic activity of the compounds of formula (I) was studied by animal pain models such as HAC writhing test, bee venom stimulating test, hot-plate test and sciatic nerve ligation test with Kunming mice and SD rats.

**Experiment example 1: Acetic acid-induced writhing test**

Kunming mice (18-22g, male and female, half each) were divided into a negative control group, an Aspirin control group, a morphine control group and a group of the compound of formula (I). Each group of mice was respectively administered the corresponding medicine (0.2ml/10g) intragastrically or subcutaneously. The mice of the negative control group were administered the same volume vehicle. After 60 minutes (intragastrically) or 30 minutes (subcutaneously), each group of mice was administered 0.6% acetic acid solution (0.4ml) by intraperitoneal injection, and then writhing times in 15 minutes were observed and recorded immediately. Writhing inhibition rate in mice was used as the pharmacodynamic index of analgesic activity.

Writhing inhibition rate (%) =

(writhing times of negative control group - writhing times of medicine group)/ Writhing times of negative control group ×100%

$ED_{50}$ values were calculated according to the result. The results are shown in Table 2.

Table 2: Analgesic activity of the compounds in writhing test

| Sample | $ED_{50}$ (mg/Kg) | |
|---|---|---|
| | intragastrically | subcutaneously (S. C) |
| Example 1 | 20 | 15 |
| Example 2 | 5 | 2 |
| Example 3 | 30 | 20 |
| Example 4 | 80 | 36 |
| Example 5 | 50 | 18 |
| Example 7 | 60 | 20 |
| Example 8 | 120 | 80 |
| Example 9 | 3 | 2 |
| Example 10 | 5 | 10 |
| Example 11 | 200 | 120 |
| Example 12 | 180 | 100 |
| Example 14 | 152 | 78 |
| Example 16 | 132 | 100 |
| Aspirin | 100 | 80 |
| Morphine | 4 | 1 |

The results of Table 2 show that the compound of formula (I) can significantly inhibit the writhing times, improve clinical symptoms and have a good analgesic effect.

**Experiment example 2: Hot-plate test in mice**

Hot-plate test is one of the conventional physiological pain models. Mice lick hind paws, jump up and so on when they are subject to thermal stimulus. Analgesics that take effect on the central nervous system can increase the thermo-pain threshold and prolong the latency of protective response.

KM female mice weighing 18-22g were employed in the test. Primary screening was done before the test to remove the animals whose responses were too slow (>30s) or too sensitive (<6s). The mice passing the primary screening were divided into a negative control group, a morphine control group and a group of the compound of formula (I). Each group of mice was respectively administered the corresponding dose of medicine intragastrically or by intravenous injection, and 60 minutes later, a hot-plate test was done on those mice. The time of first licking of the hind paws and jumping up was recorded (the data over 60s is recorded as 60) and compared with the latency of the animals in the control group. The analgesic activity of each dose was calculated and $ED_{50}$ was calculated from the analgesic activity. The results are shown in Table 3.

Table 3: Analgesic activity of the compounds in the hot-plate test

| Sample | $ED_{50}$(mg/Kg) | |
|---|---|---|
| | intragastrically | intravenous injection |
| Example 1 | 25 | 8 |
| Example 3 | 50 | 19 |
| Example 5 | 55 | 20 |
| Example 7 | 60 | 20 |
| Example 8 | 120 | 40 |
| Example 9 | 20 | 5 |
| Example 10 | 30 | 10 |

(continued)

| Sample | ED$_{50}$(mg/Kg) | |
|---|---|---|
| | intragastrically | intravenous injection |
| Example 11 | 150 | 60 |
| Morphine | 2 | 1 |

It can be seen from the results in Table 3 that in the hot-plate test, the compounds of formula (I) showed good analgesic effects.

**Experimental example 3: Bee venom test**

Adult male Sprague-Dawley (SD) rats weighing 200- 250 g were used in the test after acclimation by feeding for 1 week. First, tubes were placed intrathecally (i.t.) in rats. The rats with good conditions after 3-4 days were selected for the bee venom test. The animals were divided into an intrathecal DMSO group, an intrathecal morphine group and a group of intrathecal compounds of formula (I). 5 minutes after administration, each group of rats was intravenously injected with 50 $\mu$l of bee venom at their left hind limbs. Times of feet spontaneously drawing back every 5 minutes in 60 minutes, as well as total times of feet drawing back in an hour were observed and recorded. The response latency to heat stimulus and threshold of mechanical stimulus on both thenars of each group of rats were measured before intrathecal administration and 2 hours after bee venom injection respectively. The results are shown in Table 4.

Table 4: Analgesic activity of the compounds in the bee venom test

| Sample | ED$_{50}$ ($\mu$g/Kg) |
|---|---|
| | intrathecally |
| Example 2 | 100 |
| Example 5 | 60 |
| Example 7 | 80 |
| Example 8 | 120 |
| Example 9 | 20 |
| Example 10 | 30 |
| Example 11 | 200 |
| morphine | 5 |

It can be seen from the results shown in Table 4 that the intrathecal injection of the compounds of formula (I) can significantly reduce the total times of rats' feet spontaneously drawing back ($P<0.05$), and inhibit the spontaneous pain responses induced by bee venom markedly with the inhibition rate of 33.14% and 35.56% respectively. It can be seen from the observations that the effect of easing pain was the best at half an hour after administration; the heat response latency of rats can be prolonged significantly ($P < 0.05$); the pain threshold to heat was increased, while no obvious effect was observed on the pain sensitivity to mechanical stimulus.

**Experimental example 4: The spared nerve injury (SNI) test**

Neuropathic pain is also called, for abbreviation, neuralgia, which is a common chronic pain. It is an abnormal pain condition caused by injury or disease-induced trauma of peripheral or central nerve, and cannot be cured effectively to date. Sciatic nerve branches selective injury model (spared nerve injury, SNI) is a neuralgia model established in 2000 (see Spared nerve injury: an animal model of persistent peripheral neuropathic pain, Pain, 2000 Aug; 87(2): 149-58). The model can produce reduced robust mechanical allodynia, so it can simulate the clinic nerve pathologic pain relatively well.

Adult male Sprague-Dawley (SD) rats weighing 200-250g (Experimental Animal Center of Xuzhou Medical College) were employed in the test after acclimation by feeding for 1 week. First, Sciatic nerve branches selective injury model (spared nerve injury, SND was replicated. The rats of successful modeling were administered intragastrically on

Day 12, 13, 14 after surgery. The mechanical allodynia were measured at 60 minutes after administration every day (von Frey Cilia Apparatus, manufactured by Institute of Biology Bioengineering, Chinese Academy of Medical Science). The results are shown in Table 5.

Table 5: Analgesic activity of the compounds in the spared nerve injury (SNI) test

| Sample | $ED_{50}$ (mg/Kg) |
|---|---|
| | intragastrically |
| Example 1 | 40 |
| Example 2 | 15 |
| Example 3 | 30 |
| Example 4 | 100 |
| Example 5 | 80 |
| Example 7 | 60 |
| Example 8 | 150 |
| Example 9 | 10 |
| Example 10 | 15 |
| Example 11 | 180 |
| Example 12 | 200 |
| Example 14 | 152 |
| Example 16 | 167 |
| Gabapentin | 50 |

It can be seen from the results in Table that the mechanical threshold of each group of rats was reduced significantly on Day 7, indicating that the SNI model can produce mechanical pain sensitivity in rats and the compounds of formula (I) can increase the pain threshold of the rats significantly ($P < 0.01$). Therefore, the compounds have a better pain-easing effect in such a dose.

## Claims

1. Use of aryl piperazines of formula (I) or pharmaceutically acceptable salts thereof for the manufacture of a medicament for treating acute pain, neuropathic pain or receptive nociceptive pain in mammals, including human beings:

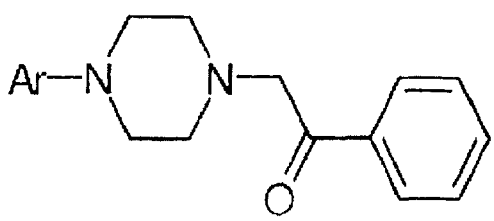

I

wherein, Ar is a phenyl monosubstituted or disubstituted with F, Cl, Br, methyl, methoxyl or trifluoromethyl; or is an unsubstituted, monosubstituted or disubstituted heteroaryl, wherein the heteroaryl is selected from benzothiazolyl, pyridyl or pyrimidinyl and the substituent(s) are selected from the group consisting of halogen, $C_{1-6}$ alkyl, $C_{1-4}$ alkoxyl and halogenated $C_{1-4}$ alkyl.

2. The compound of formula (I) or pharmaceutically acceptable salts thereof as defined in claim 1, for use in the

treatment of acute pain, neuropathic pain or receptive nociceptive pain in mammals, including human beings.

**3.** The use according to claim 1 or the compound for use according to claim 2, wherein the substituents of the heteroaryl are selected from F, Cl, Br, methyl, methoxyl and trifluoromethyl.

**4.** The use according to claim 1 or the compound for use according to claim 2, wherein the compound is selected from the group consisting of:

1) 2-[4-(2-methoxylphenyl)-piperazin-1-yl]-1-acetophenone
2) 2-[4-(3-methoxylphenyl)-piperazin-1-yl]-1-acetophenone
3) 2-[4-(4-methoxylphenyl)-piperazin-1-yl]-1-acetophenone
4) 2-[4-(2-chlorophenyl)-piperazin-1-yl]-1-acetophenone
5) 2-[4-(3-chlorophenyl)-piperazin-1-yl]-1-acetophenone
6) 2-[-4-(4-chlorophenyl)-piperazin-1-yl]-1-acetophenone
7) 2-[4-(2,3-dichlorophenyl)-piperazin-1-yl]-1-acetophenone
8) 2-[4-(4-fluorophenyl)-piperazin-1-yl]-1-acetophenone
9) 2-[4-(3-trifluoromethylphenyl)-piperazin-1-yl]-1-acetophenone
10) 2-[4-(2,3-dimethylphenyl)-piperazin-1-yl]-1-acetophenone
11) 2-[4-(6-methoxyl-2-benzothiazolyl)-piperazin-1-yl]-1-acetophenone
12) 2-[4-(6-methy)-2-benzothiazolyl)-piperazin-1-yl]-1-acetophenone
13) 2-[4-(4-methyl-2-benzothiazolyl)-piperazin-1-yl]-1-acetophenone
14) 2-[4-(6-chloro-2-benzothiazolyl)-piperazin-1-yl]-1-acetophenone
15) 2-[4-(4-chloro-2-benzothiazolyl)-piperazin-1-yl]-1-acetophenone
16) 2-[4-(2-pyrimidinyl)-piperazin-1-yl]-1-acetophenone
17) 2-[4-(6-chloro-2-pyridyl)-piperazin-1-yl]-1-acetophenone
18) 2-[4-(3-methyl pyridyl)-piperazin-1-yl]-1-acetophenone, and
19) 2-[4-(2-methyl pyridyl)-piperazin-1-yl]-1-acetophenone.

**5.** The use according to claim 1 or the compound for use according to claim 2. wherein the compound of formula (I) or pharmaceutically acceptable salts thereof is to be administrated in combination with another conventional analgesic.

**Patentansprüche**

**1.** Verwendung von Arylpiperazinen der Formel (I) oder pharmazeutisch verträglicher Salze davon zur Herstellung eines Medikaments zur Behandlung von akutem Schmerz, neuropathischem Schmerz oder rezeptivem nozizeptivem Schmerz in Säugetieren, einschließlich Menschen:

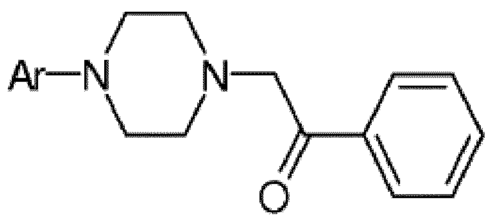

I

wobei Ar ein mit F, Cl, Br, Methyl, Methoxy oder Trifluormethyl monosubstituiertes oder disubstituiertes Phenyl ist; oder es ist ein unsubstituiertes, monosubstituiertes oder disubstituiertes Heteroaryl, wobei das Heteroaryl ausgewählt ist aus Benzothiazolyl, Pyridyl oder Pyrimidinyl und die Substituent(en) sind ausgewählt aus der Gruppe bestehend aus Halogen, $C_{1-6}$ - Alkyl, $C_{1-4}$ - Alkoxyl und halogenierten $C_{1-4}$ - Alkyl.

**2.** Verbindung der Formel (I) oder pharmazeutisch verträgliche Salze davon, wie in Anspruch 1 definiert, zur Verwendung bei der Behandlung von akutem Schmerz, neuropathischem Schmerz oder rezeptivem nozizeptivem Schmerz in Säugetieren, einschließlich Menschen.

3. Verwendung gemäß Anspruch 1 oder die Verbindung zur Verwendung gemäß Anspruch 2, wobei die Substituenten des Heteroaryls ausgewählt sind aus F, Cl, Br, Methyl, Methoxy und Trifluormethyl.

4. Verwendung gemäß Anspruch 1 oder die Verbindung zur Verwendung gemäß Anspruch 2, wobei die Verbindung ausgewählt ist aus der Gruppe bestehend aus:

1) 2-[4-(2-Methoxyphenyl)-piperazin-1-yl]-1-acetophenon
2) 2-[4-(3-Methoxyphenyl)-piperazin-1-yl]-1-acetophenon
3) 2-[4-(4-Methoxyphenyl)-piperazin-1-yl]-1-acetophenon
4) 2-[4-(2-Chlorphenyl)-piperazin-1-yl]-1-acetophenon
5) 2-[4-(3- Chlorphenyl)-piperazin-1-yl]-1-acetophenon
6) 2-[4-(4- Chlorphenyl)-piperazin-1-yl]-1-acetophenon
7)2-[4-(2,3-Dichlorphenyl)-piperazin-1-yl]-1-acetophenon
8) 2-[4-(4-Fluorphenyl)-piperazin-1-yl]-1-acetophenon
9) 2-[4-(3-Trifluormethylphenyl)-piperazin-1-yl]-1-acetophenon
10) 2-[4-(2,3-Dimethylphenyl)-piperazin-1-yl]-1-acetophenon
11) 2-[4-(6-Methoxy-2-benzothiazolyl)-piperazin-1-yl]-1-acetophenon
12) 2-[4-(6-Methyl-2-benzothiazolyl)-piperazin-1-yl]-1-acetophenon
13) 2-[4-(4-Methyl-2-benzothiazolyl)-piperazin-1-yl]-1-acetophenon
14) 2-[4-(6-Chlor-2-benzothiazolyl)-piperazin-1-yl]-1-acetophenon
15) 2-[4-(4-Chlor-2-benzothiazolyl)-piperazin-1-yl]-1-acetophenon
16) 2-[4-(2-Pyrimidinyl)-piperazin-1-yl]-1-acetophenon
17) 2-[4-(6-Chlor-2-pyridyl)-piperazin-1-yl]-1-acetophenon
18) 2-[4-(3-Methylpyridyl)-piperazin-1-yl]-1-acetophenon, und
19) 2-[4-(2-Methylpyridyl)-piperazin-1-yl]-1-acetophenon.

5. Verwendung gemäß Anspruch 1 oder die Verbindung zur Verwendung gemäß Anspruch 2, wobei die Verbindung der Formel (I) oder pharmazeutisch verträgliche Salze davon in Kombination mit einem anderen herkömmlichen Analgetikum zu verabreichen ist.

## Revendications

1. Utilisation d'aryl pipérazines de formule (I) ou de sels pharmaceutiquement acceptables de celles-ci pour la fabrication d'un médicament destiné au traitement de la douleur aiguë, de la douleur neuropathique ou de la douleur nociceptive réceptive chez les mammifères, notamment les êtres humains :

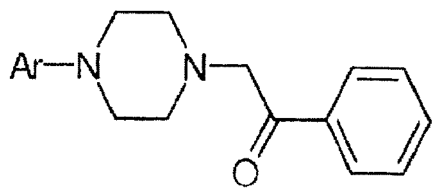

I

formule dans laquelle Ar représente un groupe phényle monosubstitué ou disubstitué par un atome de F, Cl, Br ou un groupe méthyle, méthoxyle ou trifluorométhyle ; ou représente un groupe hétéroaryle non substitué, monosubstitué ou disubstitué, dans lequel le groupe hétéroaryle est choisi parmi un groupe benzothiazolyle, pyridyle ou pyrimidinyle et le(s) substituant(s) est (sont) choisi(s) dans le groupe constitué d'un atome d'halogène et des groupes alkyle en $C_1$ à $C_6$, alcoxyle en $C_1$ à $C_4$ et alkyle halogéné en $C_1$ à $C_4$.

2. Composé de formule (I) ou sels pharmaceutiquement acceptables de celui-ci tels que définis dans la revendication 1, à utiliser dans le traitement de la douleur aiguë, de la douleur neuropathique ou de la douleur nociceptive réceptive chez les mammifères, notamment les êtres humains.

3. Utilisation selon la revendication 1 ou composé à utiliser selon la revendication 2, les substituants du groupe hétéroaryle étant choisis parmi les atomes de F, Cl, Br et les groupes méthyle, méthoxyle et trifluorométhyle.

**4.** Utilisation selon la revendication 1 ou composé à utiliser selon la revendication 2, le composé étant choisi dans le groupe constitué de :

1) 2-[4-(2-méthoxylphényl)-pipérazin-1-yl]-1-acétophénone
2) 2-[4-(3-méthoxylphényl)-pipérazin-1-yl]-1-acétophénone
3) 2-[4-(4-méthoxylphényl)-pipérazin-1-yl]-1-acétophénone
4) 2-[4-(2-chlorophényl)-pipérazin-1-yl]-1-acétophénone
5) 2-[4-(3-chlorophényl)-pipérazin-1-yl]-1-acétophénone
6) 2-[4-(4-chlorophényl)-pipérazin-1-yl]-1-acétophénone
7) 2-[4-(2,3-dichlorophényl)-pipérazin-1-yl]-1-acétophénone
8) 2-[4-(4-fluorophényl)-pipérazin-1-yl]-1-acétophénone
9) 2-[4-(3-trifluorométhylphényl)-pipérazin-1-yl]-1-acétophénone
10) 2-[4-(2,3-diméthylphényl)-pipérazin-1-yl]-1-acétophénone
11) 2-[4-(6-méthoxyl-2-benzothiazolyl)-pipérazin-1-yl]-1-acétophénone
12) 2-[4-(6-méthyl-2-benzothiazolyl)-pipérazin-1-yl]-1-acétophénone
13) 2-[4-(4-méthyl-2-benzothiazolyl)-pipérazin-1-yl]-1-acétophénone
14) 2-[4-(6-chloro-2-benzothiazolyl)-pipérazin-1-yl]-1-acétophénone
15) 2-[4-(4-chloro-2-benzothiazolyl)-pipérazin-1-yl]-1-acétophénone
16) 2-[4-(2-pyrimidinyl)-pipérazin-1-yl]-1-acétophénone
17) 2-[4-(6-chloro-2-pyridyl)-pipérazin-1-yl]-1-acétophénone
18) 2-[4-(3-méthylpyridyl)-pipérazin-1-yl]-1-acétophénone, et
19) 2-[4-(2-méthylpyridyl)-pipérazin-1-yl]-1-acétophénone.

**5.** Utilisation selon la revendication 1 ou composé à utiliser selon la revendication 2, le composé de formule (I) ou les sels pharmaceutiquement acceptables de celui-ci devant être administrés en association avec un autre analgésique classique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1627871 A
- EP 1123926 A
- WO 2007071055 A
- WO 2007104783 A

### Non-patent literature cited in the description

- **LI, J. et al.** Design and synthesis of aralkyl-ketone piperazine derivatives and their antalgic activities. *Acta Pharmaceutica Sinica,* 2007, vol. 42 (11), 1166-1175
- Spared nerve injury: an animal model of persistent peripheral neuropathic pain. *Pain,* August 2000, vol. 87 (2), 149-58